# EUROPEAN PATENT APPLICATION

(11) **EP 3 778 637 A1**
(43) Date of publication of application: **17.02.2021**
(21) Application number: 19776776.7
(22) Date of filing: 29.03.2019
(51) Int. Cl.: C07K 16/28, C12N 5/18, G01N 33/53

(54) **MONOCLONAL ANTIBODY SPECIFICALLY REACTING WITH DUPAN-2 ANTIGEN AND METHOD FOR PRODUCING SAME**

(30) Priority: 30.03.2018 JP 2018069050; 25.05.2018 JP 2018100969; 05.09.2018 CN 201811030796
(71) Applicant: Sekisui Medical Co., Ltd., Tokyo 103-0027 (JP)
(72) Inventor: MIURA Tomohiro, Tokyo 103-0027 (JP); MIYAZAKI Osamu, Tokyo 103-0027 (JP)
(74) Representative: Hoffmann Eitle
(86) International application number: PCT/JP2019/014321
(87) International publication number: WO 2019/189874

(57) **Abstract**

The antigen specificity of the antibody that recognizes a glycan antigen and is used as a tumor marker is not sufficient, and it has been a drawback that tumor marker detection using such an antibody is low in detection specificity, thereby deteriorating accuracy of cancer diagnosis.

To solve the object described above, the present invention provides an antibody specifically reactive with a glycan of DUPAN-2 antigen for use as a tumor marker, and a production method thereof.

## Description

### Technical Field

The present invention relates to a monoclonal antibody specifically reactive with DUPAN-2 antigen, and a production method thereof.

### Background Art

Recently, a lot of antibodies with respect to glycan antigen used as tumor markers have been reported. It is known that DUPAN-2, a monoclonal antibody prepared using human pancreatic cancer culture cell HPAF-1 as an immune antigen is reactive with a mucin-like protein that is produced by pancreatic cancer cells with a high possibility, and it is considered that sialic acid relates to an antigen-determining group thereof (Non-Patent Literatures 1 and 2). The antigen is called DUPAN-2 antigen and has been used as a marker especially for digestive cancers such as pancreatic cancer and biliary cancer.

Moreover, NCC-ST-439 antigen that an antibody recognizing esophageal cancer tissue is a glycan antigen containing sialic acid, and it is known that NCC-ST-439 antigen increases in case of stomach cancer, lung cancer, breast cancer, and pancreatic cancer (Non-Patent Literature 3) .

On the other hand, the antibody that recognizes a glycan antigen and is used as a tumor marker for these cancers is isolated in general as an antibody that recognizes certain cancer cells as a whole, and is not sufficient in terms of antigen specificity, so that this antigen is reactive with a plurality of glycan antigen. It has been a drawback that tumor marker detection using such an antibody is low in detection specificity, thereby deteriorating accuracy of cancer diagnosis.

Therefore, for the sake of more accurate diagnosis using a tumor marker, it has been a demand for an antibody specifically reactive with a particular glycan antigen.

### Citation List

### Non-Patent Literatures

Non-Patent Literature 1: Rinsho Byori (clinicopathology), 1986, Vol. 34, No. 6, pp. 705-710
Non-Patent Literature 2: Gan to Kagaku Ryoho (Cancer and Chemical Therapy), 1986, Vol. 13, No. 11, pp. 3207-3214
Non-Patent Literature 3: GANN Japanese Journal of Cancer Research, 1984, Vol. 75, No. 6, pp. 485-488

### Summary of Invention

### Technical Problem

An object of the present invention is to provide an antibody specifically reactive with a glycan of DUPAN-2 antigen for use as a tumor marker, and a production method thereof.

### Solution to Problem

The present inventors diligently studied to solve the problem, and, by using a glycan of DUPAN-2 antigen itself as an antigen, successfully obtained such an antibody that specifically recognizes this antigen. The antibody thus obtained by the method recognizes the DUPAN-2 antigen highly specifically among mucin antigens used as various tumor markers, and does not react with the other tumor marker antigens such as NCC-ST-439.

Therefore, the present invention provides the followings according to exemplary aspects thereof.
[1] An antibody or an antigen-binding fragment thereof,
   which reacts with DUPAN-2 antigen having a glycan structure below:

      **Neu5Acα₂-3Galβ1-3GlcNAcβ1--3Gal-**

      and
   does not react with NCC-ST-439 antigen having a glycan structure below: 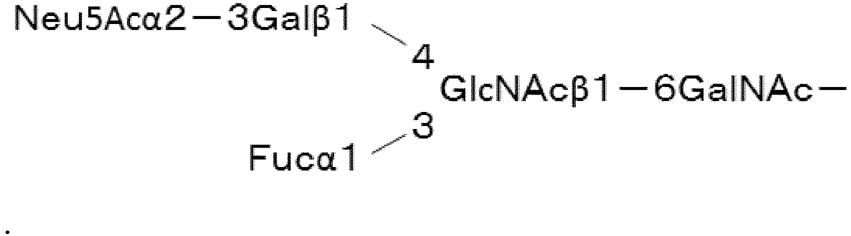
[2] The antibody or the antigen-binding fragment thereof according to [1], in which reactivity against NCC-ST-439 antigen is less than 10% of reactivity against DUPAN-2 antigen.
[3] The antibody or the antigen-binding fragment thereof according to [1], in which reactivity against NCC-ST-439 antigen is less than 1% of reactivity against DUPAN-2 antigen.
[4] The antibody or the antigen-binding fragment thereof according to any one of [1] to [3], being a rat antibody.
[5] A method of producing the antibody or the antigen-binding fragment thereof according to any one of [1] to [4], the method including:
   a step of inoculating an animal with a polymer compound bound with DUPAN-2 antigen.
[6] A method of producing cells for producing the antibody or the antigen-binding fragment thereof according to any one of [1] to [4], the method including:
   a step of inoculating an animal with a polymer compound bound with DUPAN-2 antigen.
[7] The method according to [5] or [6], in which the polymer compound bound with DUPAN-2 antigen does not contain a peptide portion of DUPAN-2 antigen-binding peptide.
[8] An immunity measuring method, in which the antibody or the antigen-binding fragment thereof according to any one of [1] to [3] is employed.
[9] An immunity measuring reagent including the antibody or the antigen-binding fragment thereof according to any one of [1] to [3].

### Advantageous Effects of Invention

The antibody of the present invention highly specifically recognizes the DUPAN-2 antigen among mucin antigens used as various tumor markers, and does not react with the other tumor marker antigens such as NCC-ST-439. Thus, with this antibody, it becomes possible to specifically detect cancer cells that express DUPAN-2 antigen, such as pancreatic cancer cells, thereby making it possible to perform more accurate cancer diagnosis.

Moreover, by using a method of the present invention for preparing an antibody that specifically recognizes a mucin antigen for use as a tumor markers, it becomes possible to obtain an antibody that highly specifically reacts with a particular tumor marker.

### Brief Description of Drawings

Fig. 1 is a view schematically illustrating a method of producing an antibody that specifically recognizes DUPAN-2 antigen.
Fig. 2 is a view illustrating an experiment result of antigen-immobilized ELISA method.
Fig. 3 is a view illustrating a result of epitope analysis of a monoclonal antibody of the present invention.
Fig. 4 is a view illustrating a result of specificity analysis of a monoclonal antibody of the present invention.
Fig. 5a is a view illustrating reactivity of the monoclonal antibody of the present invention against DUPAN-22 Standard product.
Fig. 5b is a view illustrating correlation between absorbance of the monoclonal antibody of the present invention in an experiment using cancer patient blood serum and a value of DUPAN-2 measured using a conventional ELISA kit.

### Description of Embodiments

In the followings, embodiments of the present invention will be described. It should be understood that the following explanations are merely for illustrative purposes and the scope of the present invention is not limited by these explanations and can be put into practice in a manner modified as appropriate within the gist of the present invention.

### (Definitions)

Unless otherwise specified, all technical and scientific terms used in this specification have the meanings as understood by the person skilled in the art to which the present invention pertains.

In this specification, in case where a plurality of numerical ranges is presented, it means that ranges between arbitrary combinations of a lower limit of one of the plurality of numerical ranges and an upper limit of another one of the plurality of numerical ranges are also encompassed.

In this specification, expressions an antibody "reacts," is reactive," "has reactivity," or "bonds" with a compound, or an antibody "recognizes" a compound encompass meanings usually used in the field to which the present invention pertains, and these expressions are synonymously usable. Confirmation as to whether or not an antibody "reacts" with a compound can be carried out by techniques well-known to a person skilled in the art such as an antigen-immobilized ELISA method, a competitive ELISA method, and a sandwich ELISA method, while the confirmation can be performed by a method using the principal of the surface plasmon resonance (SPR method) or the like method. The SPR method can be carried out by using a device, a sensor, and reagents, which are commercially available under the name of Biacore (registered trademark).

In this specification, what is meant by a phase an antibody according to the present invention "does not react" with a compound is that the antibody according to the present invention does not substantially react with the compound. What is meant by the expression "not substantially reacting" is that, for example, in the antigen-immobilized ELISA method, the addition of the compound does not substantially affect the bonding between the antibody and the immobilized antigen. The "not substantially reacting" can be confirmed also by a method or means well-known for a person skilled in the art other than the antigen-immobilized ELISA method.

In this specification, what is meant by the phase "an antibody "specifically reacts" or the word "specificity" of an antibody is an ability of reacting with an epitope presented on the antigen in such a manner that the antibody can be detected but reactivity with the other antigens is relatively limitedly detectable or substantially not detectable. For example, in case where an antibody "specifically reacts" with a certain antigen, the antibody reacts with the antigen but not with the other antigens. In preferable aspects, in case where an antibody "specifically reacts" with a certain antigen, for example, interaction between the antigen immobilized in the antigen-immobilized ELISA method and the antibody is hindered by the antigen being free but not by the other antigens being free. For example, in case where the hindering caused by the antigen-immobilized ELISA method is represented by IC₅₀ of free antigen, IC₅₀ of the non-specific antigen may be, compared with IC₅₀ of the specific antigen, 10 times, 100 times, 200 times, 300 times, 400 times, 500 times, 1000 times, or 10000 times. Moreover, in case where IC₅₀ of the specific antigen is 1/X of that of another antigen, the reactivity of the specific antigen may be expressed as being X times of reactivity of another antigen. It is preferable that reactivity of another antigen be less than 20%, 15%, 10%, 5%, 4%, 3%, 2%, or 1% of the specific antigen. In preferable aspects of the present invention, the antibody of the present invention reacts with DUPAN-2 antigen but not with NCC-ST-439 antigen.

In this specification, what is meant by the "antibody" is an immunoglobulin molecule including two heavy chains (H) and two light chains (L) connected with each other via four polypeptide chains and disulfide bonding. Each of the heavy chains includes a heavy chain variable region ("HCVR" or "VH") and a heavy chain constant region (including CH₁, CH₂, and CH₃ domains) . Each of the light chains includes a light chain variable region ("LCVR" or "VL") and a light chain constant region (CL). The VH and VL regions may further be divided into hypervariable regions named as complementary-determining regions (CDR), which are scattered within regions named as frameworks (FR) where many of them can be stored. Each VH and VL includes three CDRs and four FRs, and is arranged from the amine terminal to the carboxy terminal in the following order: FR1, CDR1, FR2, CDR2, FR3, CDR3, and FR4. The variable regions of the heavy chains and the light chains include a binding domain. The term "antibody" also includes all genetically recombinant antibodies of the antibody, for example, antibodies expressed in prokaryote and antibodies not glycosylated.

Moreover, as described in Padlan (1995 FASEB J. 9:133-139), Vajdos et al. 2002 J Mol Biol 320:415-428, it is known that only some of the CDR residues contacts with the antigen in reality. CDR residues not in contact with the antigen can be identified by molecular modeling or by experience from CDR regions of Kabat being present outside CDR of Chothia. In case where CDR or one or more of residues thereof are removed, the CDR or one or more of residues thereof are substituted in general with another human antibody sequence or an amino acid that occupies a corresponding site in consensus of such a sequence. Where to substitute in the CDR and the amino acid can be also selected by experience. The experiential substitution may be conservative substitution or nonconservative substitution.

In this specification, what is meant by an "antigen-binding fragment" of an antibody is one or more fragments of the antibody, which maintain the ability of specifically binding with the antigen (for example, DUPAN-2). Non-restrictive examples of such binding fragments encompassed by the "antigen-binding fragment" of an antibody include: (i) Fab fragment, which is a monovalent fragment including VL, VH, CL, and CH domains; (ii) F(ab')₂ fragment, which is a divalent fragment including two Fab fragments bonded via a disulfide bridge at a hinge region; (iii) Fd fragment including VH and CH domains; (iv) Fv fragment including VL and VH domains of a single arm of an antibody; (v) dAb fragment including a VH domain (Ward et al., (1989) Nature 341:544-546); (vi) isolated complementary-determining region (CDR); and (vii) combinations of two or more isolated CDRs, which may be linked via a synthetic linker. Moreover, such binding fragments may be produced as a single protein chain, as being known as a single chain Fv (scFv), in which VL and VH regions are formed in pair by using a synthetic linker with the use of gene recombination method (Bird et al. (1988) Science 242: 423-426; and Huston et al. (1988) Proc. Natl. Acad. Sci. USA 85: 5879-5883). Moreover, the "antigen-binding fragment" may be binding domain immunoglobulin fused protein including: (i) a binding domain polypeptide fused with an immunoglobulin hinge region polypeptide; (ii) an immunoglobulin heavy chain CH2 constant region fused with a hinge region; and (iii) an immunoglobulin heavy chain CH3 constant region fused with a CH2 constant region. These antibody fragments may be obtained by a conventional technique well-known to a person skilled in the art.

The antibody usable in the present invention or an antigen-binding fragment thereof may be derived from any animal origin including birds and mammals. Preferably, the antibody or fragment may be derived from human, chimpanzee, rodent animal (such as mouse, rat, guinea pig, or rabbit), chicken, turkey, pig, sheep, goat, camel, cow, horse, donkey, cat, or dog origin. The antibody of the present invention encompasses chimeric molecules in which a constant region of an antibody derived from a certain species is combined with an antigen-binding site derived from another species. Furthermore, the antibody of the present invention encompasses humanized molecules in which an antigen-binding site of an antibody derived from a non-human species (such as mouse origin), a constant region derived from human origin, and a framework region.

The antibody of the present invention may be obtained from a hybridoma expressing the antibody or a host cell expressing the antibody as a result of genetic recombination. As the host cell, a CHO cell, a lymphocyte cell, a bacteria cell such as E. coli, and fungi cell such as yeast.

Moreover, the antibody of the present invention may be produced in a non-human animal or a plant, which has been subjected to gene transfer by using a gene recombination technique.

As the antibody of the present invention, for example, a monoclonal antibody produced by hybridoma S19201R is preferable. The hybridoma S19201R is internationally deposited under the Budapest Treaty as below.

The name of the depositary institution: The National Institute of Technology and Evaluation, the NITE Patent Microorganisms Depositary, The address of the depositary institution: Room 122, 2-5-8, Kazusa Kamatari, Kisarazu City, Chiba Pref., (Post code 292-0818), Accession number: NITE BP-02721, The date of deposition: May 17, 2018

In this specification, an "alkyl or alkyl group" may be an aliphatic hydrocarbon group that may be linear one, branched one, cyclic one, or a combination thereof. The number of carbons in the alkyl group is not particularly limited, but for example, may be in a range of 1 to 20 (C1 to C20), 1 to 15 (C1 to C15), or 1 to 10 (C1 to C10).

The alkyl group may have one or more arbitrary substituents. Examples of C1 to C8 alkyls include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, or the like. Examples of the substituent include alkoxy group, halogen atoms (which may be any one of fluorine atom, chlorine atom, bromine atom, or iodine atom), amino group, mono- or di-substituted amino group, substituted silyl group, acyl, and the like, but the substituent is not limited to these. In case where the alkyl group has two or more substituents, the two or more substituents may be identical with or different from each other.

In this specification, what is meant by the word "alkylene" is divalent linear or branched saturate hydrocarbon group.

In this specification, in case where a certain functional group is defined as "may be substituted," the type of substituent, the position of the substitution, and the number of substituents are not particularly limited, and if the group has two or more substituents, the two or more substituents may be identical with or different from each other. Examples of the substituent include alkyl group, alkoxy group, hydroxy group, carboxyl group, halogen atoms, sulfo group, amino group, alkoxy carbonyl group, oxo group, and the like, but the substituents are not limited to these. These substituents may have a substituent(s). Such examples include halogenated alkyl group and the like, but not limited to these.

In this specification, what is meant by "DUPAN-2 antigen" is a glycan having the following structure, which is reported as being recognizable by DUPAN-2 antibody (Kawa. S. et. al. Pancreas (1994), 9(6): 692-697)).

**Neu5Acα2-3Galβ1-3GlcNAcβ1-3Gal-**

In this specification, what is meant by "NCC-ST-439 antigen" is a glycan having the following structure, which is reported as being recognizable by NCC-ST-439 antibody (Kumamoto. K. et. al. Biochem. Biophys. Res. Commun. (1998), 247(2): 514-17).

Neu5Acα2－3Galβ1
4
GlcNAcβ1－6GalNAc－
3
Fucα1

In this specification, what is meant by "DUPAN-2 antigen-binding peptide" is a peptide to which DUPAN-2 antigen is bound, and which is found in tumor cells and the like in vivo.

### (Method of Preparing Antibody Specifically Reactive with DUPAN-2 antigen)

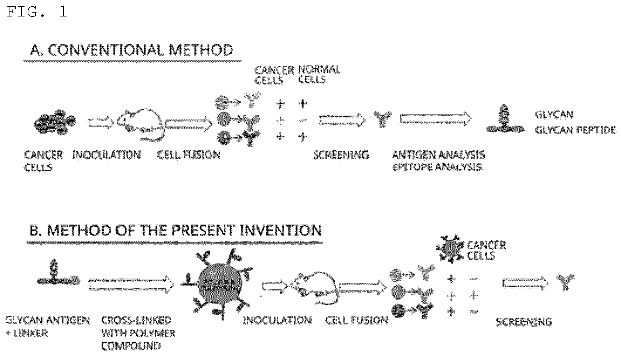

The antibody of the present invention that specifically recognizes DUPAN-2 antigen is obtained by a method schematically illustrated in Fig. 1. More specifically, compared with a conventional tumor marker reactive antibody that is isolated by using tumor cells themselves as an antigen (Fig. 1A), the method of the present invention for preparing an antibody that specifically reacts with DUPAN-2 antigen (Fig. 1B) is such that the glycan constituting DUPAN-2 antigen is caused to be carried by a polymer compound as a carrier via a linker, and the glycan on the carrier is inoculated in a mammal such as a mouse. Using a known method such as one described in Antibodies, A Laboratory Manual (Cold Spring Harbor Laboratory Press, (1988)), by removing spleen cells or lymph node cells of the animal, and the cells are fused with myeloma cells, thereby producing hybridomas. From the hybridoma cell group thus prepared, a hybridoma cell that produces an antibody specifically reactive with cancer cells is isolated.

Compared with the conventional method that requires epitope analysis after the antibody is obtained finally, the method of the present invention can efficiently produce the antibody because the immunogen and the epitope are identical with each other, and can produce an antibody that specifically reacts with a particular glycan antigen. However, the method of the present invention requires an advanced technique in glycan synthesis.

The linker is not particularly limited in terms of its structure, but may be, for example, a C1 to C12 alkyl group, alkylene group, ethylene glycol, polyethylene glycol, amino acid, peptide, or the like, which may be substituted.

The polymer compound is not particularly limited, but may be, for example, a protein such as albumin, ovalbumin, Pseudomonas aeruginosa exotoxin, tetanus toxin, ricin toxin, diphtheria toxin, cholera toxin, heat-labile enterotoxin, keyhole limpet hemocyaninklh, epidermal growth factor, fibroblast growth factor, transferrin, platelet-derived growth factor, poly-L-lysine, or poly-L-glutamine.

The polymer compound of the present invention to which DUPAN-2 antigen is bound may or may not contain part of a DUPAN-2 antigen binding peptide. In preferable aspects, the polymer compound of the present invention to which DUPAN-2 antigen is bound does not contain a DUPAN-2 antigen binding peptide part.

The preparation of the hybridoma may be performed according to a well-known method in this field, and for example, polyethylene glycol method, a method using Sendai virus, a method using an electric current, or the like may be adopted for the preparation of the hybridoma. The hybridoma thus obtain may be multiplied according to a well-known method, and it is possible to select a desired hybridoma while confirming properties of the antibody produced therefrom. Cloning of the hybridoma may be carried out by a well-known method such as, for example, a limiting dilution method or a soft agar method.

Apart from directly producing from the hybridoma, the antibody thus obtained may be obtained by gene recombination to prepare host cells expressing the antibody, and preparing the antibody from the host cells. As the host cell, a CHO cell, a lymphocyte cell, a bacteria cell such as E. coli, and fungi cell such as yeast.

It should be noted that the method of preparing the antibody is not limited to DUPAN-2 antigen, and is applicable to preparation of antibodies specific to glycan antigens of the other well-known glycan proteins. For example, with the same method, a monoclonal antibody specifically reactive with NCC-ST-439 antigen mentioned above was successfully prepared.

### (Applications of Antibody of the Present Invention)

The antibody of the present invention is applicable to an immunity measuring method for performing detection of DUPAN-2 antigen in a biological sample as detection of a tumor marker. More specifically, it is possible to adopt various well-known method for detecting a mucin tumor marker in a biological sample by using the antibody, for example, Enzyme-Linked Immunosolvent Assay (ELISA), Radiommunoassay (RIA), Immunofluorescent measurement, immune precipitation, equilibrium dialysis, immune diffusion, and the other techniques can be used, but the method of detecting is not limited to these (for example, see Harlow and Lane, Antibodies: A Laboratory Manual, Cold Spring Harbor Laboratory, 1988; Weir, D. M., Handbook of Experimental Immunology, 1986, Blackwell Scientific, Boston). In one aspect, the antibody of the present invention may be used as an immobilized (solid phase) antibody immobilized on an insoluble carrier, or a labelled antibody labelled with a labelling material. Such an immobilized antibody or a labelled antibody are also included within the scope of the present invention. For example, an immobilized antibody may be produced by physically adsorbing or chemically bonding the antibody of the present invention onto the insoluble carrier (the chemical bonding may involve an appropriate spacer). As the insoluble carrier, an insoluble carrier made from a polymer material such as polystyrene resin, an inorganic material such as glass, or polysaccharide material such as cellulose and agarose. The insoluble carrier is not limited in terms of its shape and may have an arbitrary shape such as a plate-like shape (for example, microplate or membrane), a beads or fine particle-like shape (for example, latex particles or magnetic particles), or a tubelike shape (for example, test tube).

Examples of the labelling material for producing the labelled antibody include enzymes, fluorescent materials, chemically light emitting materials, biotin, avidin, radioactive isotope, gold collide particles, color latex, and the like. As a method of binding the labelling material and the antibody, a glutaraldehyde method, a maleimide method, a pyridyl disulfide method, or a periodic acid method, or the like available for a person skilled in the art may be adopted. The immobilized antibody and the labelled antibody are not particularly limited in terms of their kinds and production methods. For example, enzymes such as peroxidase or alkali phosphatase (hereinafter, which may be referred to as ALP) may be used as the labelling material. In this case, enzyme activity can be measured by using a substrate specific to the enzyme (for example, 1,2-phenylenediamine (hereinafter, which may be referred to as OPD) or 3,3',5,5'-tetramethyl benzidine in case of a horseradish peroxidase (hereinafter, which may be referred to as HRP), or p-nitrophenyl phosphate in case of ALP). In case where biotin is used as the labelling material, it is generally arranged such that biotin is reacted with avidin or an enzyme-modified avidin.

The antibody of the present invention may be provided as an immunity measuring reagent for use in the method of immunity measuring. The reagent may further include, apart from the antibody of the present invention, the other constituent(s) necessary for carrying out the method of immunity measuring, such as a buffer solution, or a preservative.

In one aspect, for example, in case where the antibody of the present invention includes an enzyme as the labelling material, the selectively binding material of the present invention may be provided in the form of a kit together with a reagent including the substrate specific to the enzyme.

### Examples

In the followings, the present invention will be described in more details referring to Examples, but the present invention is not limited to these Examples.

### [Experiment Example 1] Production of Monoclonal Antibody Specific to DUPAN-2 Antigen

### 1. Materials

(1) DUPAN-2-modified maleimide, various glycan-related compounds
(2) Freund's Complete Adjuvant: made by Wako Pure Chemical Industries, Ltd., 014-09541
(3) Myeloma cells (SP2/O)
(4) RPMI1640, GlutaMAX: made by GIBCO, 61870-036
(5) Fetal Bovine Serum (FBS): made by BIOLOGICAL INDUSTRIES, 04-001-1A
(6) HAT medium: made by Cosmo Bio Co., Ltd. 16213008
(7) 96-well plate: NUNC, 167008
(8) HRP-labelled goat anti-rat IgG (H&L) antibody: made by Southern Biotech, 3050-05
(9) "Determiner (registered trademark) DUPAN-2" (Conventional ELISA kit; made by Kyowa Medics): including, as constituent reagents, HRP-labelled DUPAN-2 antibody (HRP-labelled conventional antibody) and DUPAN-2 Standard product.

### 2. Preparation of DUPAN-2-Modified Maleimide-Crosslinked Protein for Immunogen

For cross-linking glycan-modified maleimide, human transferrin was reduced and the reduced human transferrin and the glycan maleimide were mixed in PBS at a weight ratio of 4: 1, reacted at room temperature for 2 hours, and let stand at 4°C overnight. After that, dialysis was carried out to replace the buffer solution with PBS, thereby obtaining a conjugate solution for inoculation.

### 3. Preparation of Anti-DUPAN-2 Monoclonal Antibody-Producing Hybridoma

### (3-1) Inoculation to Animal

An emulsion prepared by mixing an equal amounts of the conjugate solution for inoculation (0.2 to 2 mg/ml) and Freund's Complete Adjuvant was injected to F344 rats in an amount in a range of 10 to 40 µg/animal. The injection of the emulsion was repeated 7 to 8 times with 1-week intervals. An antibody titer in an antiserum obtained by collecting blood from a tail vein was measured by antigen-immobilized ELISA method described later.

### (3-2) First Screening (Antigen-immobilized ELISA Method)

The presence of the anti-DUPAN-2 antibody in the antiserum of the immunized animal was confirmed by ELISA method with an immobilized conjugate solution containing DUPAN-2 and BSA prepared in the same method as the conjugate solution for inoculation (antigen-immobilized ELISA method). The antigen-immobilized ELISA method is as below.

### (3-2-1) Preparation of Plate for Antigen-Immobilized ELISA Method

A conjugate solution containing DUPAN-2 and BSA was dissolved in a 20 mM phosphate buffer solution including 150 mM sodium chloride (pH 7.2; hereinafter, which is referred to as PBS) to attain a concentration of 1 µg/mL, and 50 µL of the solution thus prepared was dispensed in each well of 96-well microplate, and left stand at room temperature for 2 hours or at 4°C overnight.

After each well was washed three times with 400 µL of PBS containing 0.05% Tween (registered trademark) 20 (hereinafter, which is referred to as PBST), 100 µL of PBST containing 1% bovine serum albumin (hereinafter, which is referred to as BSA-PBST) was added therein, and blocking was carried out at room temperature for 1 hour or at 4°C overnight. The plate thus prepared was used as a plate for ELISA.

### (3-2-2) Antigen-immobilized ELISA Method

After each well of the plate for ELISA was washed three times with 400 µL of PBST, 1500 to 13500-fold dilutions with BSA-PBST of the immunized animal antiserum and non-immunized animal serum were dispensed in each well by 50 µL, and left stand at room temperature for 1 hour. After each well was washed three times with 400 µL of PBST, a 5000-fold dilution of HRP-labelled rat IgG (H&L) diluted with BSA-PBST was inoculated in each well by 50 µL, and left stand at room temperature for 1 hour. After each well was washed three times with 400 µL of PBST, 50 µL of citrate buffer solution (pH 5.0) containing 0.2% o-phenylenediamine and 0.02% hydrogen peroxide was added therein and let stand at room temperature for 10 min. After that, 50 µL of 1.5 N sulfuric acid was added therein to stop enzyme reaction, and absorbance at wavelength of 492 nm was measured.

### (3-2-3) Experiment Result of Antigen-immobilized ELISA Method

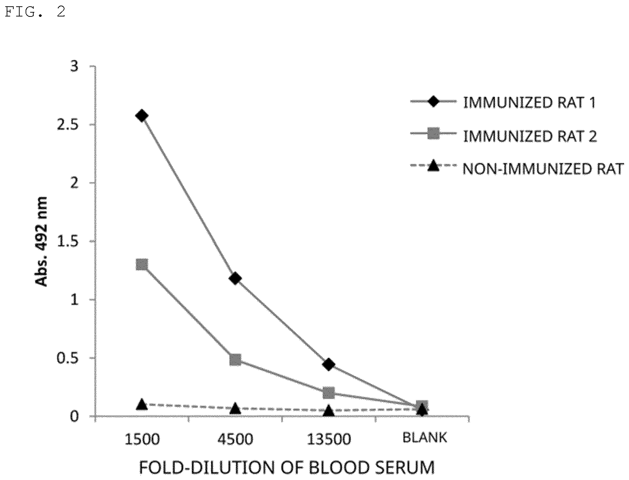

The result is shown in Fig. 2. Compared with the non-immunized animal serum, the less diluted dilutions of any immunized animal antiserum showed a higher absorbance. In this measurement system, the absorbance depends on the antibody concentration with respect to the DUPAN-2 contained in the animal serum That is, this result demonstrates that the immunized animal antiserum contained a greater concentration of the antibody with respect to the DUPAN-2 compared with the non-immunized animal serum, thereby having a greater antibody titer. From rats having a high antibody titer as the result of the measurement, a spleen or a lymph node was removed and spleen-derived cells or lymph-node-derived cells were prepared and used for cell fusion.

### (3-3) Cell Fusion

Either the spleen-derived cells or the lymph node-derived cells were mixed with myeloma cells at a cell number ratio of 1 : 1, and electrofused. The cells thus fused were dispersed in a HAT medium and incubated at 37°C in 5% CO₂ for 8 days in a CO₂ incubator, thereby obtaining fused cells (hybridoma).

### (3-4) Selection of Hybridoma (Antigen-immobilized ELISA Method)

A similar antigen-immobilized ELISA method was carried out except that, instead of the immunized animal antiserum, a culture supernatant of the fused cells was used. As a result of the measurement, wells showed a higher absorbance was selected as a well in which the anti-DUPAN-2 antibody-producing hybridoma was present (positive well).

### (3-5) Cloning

Using the anti-DUPAN-2 antibody-producing strain hybridoma selected via the first screening and second screening, monocloning of the hybridoma and purification of monoclonal antibody were carried out.

The monocloning was carried out by a usual method (Limiting Dilution Method), and positive wells were selected by the same method as the antigen-immobilized ELISA method, and finally one type of anti-DUPAN-2 monoclonal antibody-producing hybridoma. The monoclonal antibody produced by the hybridoma S19201R is referred to as S19201R antibody herein.

### [Experiment Example 2] Epitope Analysis of Monoclonal Antibody of the Present Invention

### 1. Experiment Method

Whether or not the epitope of DUPAN-2 reactive with S19201R antibody was similar to the conventional DUPAN-2 antibody was confirmed by competitive ELISA described later. To being with, a plate for ELISA was prepared by the same method as for the antigen-immobilized ELISA method described above. Moreover, using BSA-PBST as a solvent, 2-fold dilution of HRP-labelled conventional antibody and 2 to 8-fold dilutions of a culture supernatant of S19201R antibody-producing hybridoma, or a culture supernatant of hybridoma producing a control antibody not reactive with DUPAN-2 were mixed together. Thereby mixture solutions were obtained. After each well of the plate for ELISA was washed with 400 µL of PBST three times, the mixture solutions were dispensed in the wells by 50 µL/well, and left stand at room temperature for 1 hour.

After each well was washed three times with 400 µL of PBST, 50 µL of citrate buffer solution (pH 5.0) containing 0.2% o-phenylenediamine and 0.02% hydrogen peroxide was added therein and left stand at room temperature for 10 min. After that, 50 µL of 1.5 N sulfuric acid was added therein to stop enzyme reaction, and absorbance at wavelength of 492 nm was measured.

### 2. Experiment Result

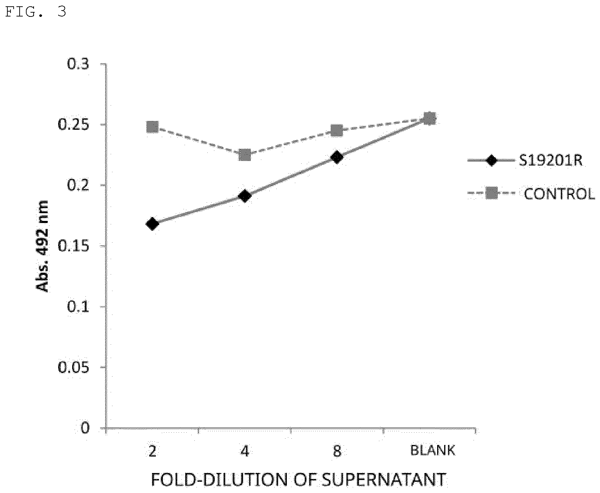

Results are shown in Fig. 3. In case where the control antibody and the HRP-labelled conventional antibody were mixed together, no decrease in absorbance was observed. However, in case where S19201R antibody and the HRP-labelled conventional antibody were mixed together, a decrease in absorbance was observed. In this measurement system, the absorbance depends on the amount of the HRP-labelled conventional antibody bound with the conjugate of DUPAN-2 immobilized on the plate and BSA. That is, a decrease in absorbance as a result of the addition of S19201R antibody indicates that the S19201R antibody in the solution binds with the vicinity of binding site of the HRP-labelled conventional antibody and the conjugate, thereby hindering the binding of the HRP-labelled conventional antibody with the conjugate. Thus, this demonstrates that the S19201R antibody recognizes the epitope similar to the conventional DUPAN-2 antibody.

### [Experiment Example 3] Specificity Analysis of Monoclonal Antibody of the Present Invention

### 1. Experiment Method

The specificity of S19201R antibody was confirmed by the competitive ELISA described later. To being with, a plate for ELISA was prepared by the same method as for the antigen-immobilized ELISA method described above. Moreover, using BSA-PBST as a solvent, an 8-fold dilution of the culture supernatant of S19201R antibody-producing hybridoma and a 2-fold dilution of the HRP-labelled conventional antibody were mixed with a conjugate of NCC-ST-439 glycopeptide and BSA, a conjugate of DUPAN-2 glycomaleimide and BSA, or purified glycan Sialyl Lewis X (sLex), which were adjusted to 0.1 to 10 µg/mL. Thereby mixture solutions were obtained. After each well of the plate for ELISA was washed with 400 µL of PBST three times, the mixture solutions of the culture supernatant of S19201R antibody-producing hybridoma and the glycan-related compounds were dispensed into the wells by 50 µL/well and left stand at room temperature for one hour. Into the other wells, BSA-PBST was dispensed by 50 µL/well and left stand at room temperature for one hour. Among the wells, the wells in which the mixture solutions of the culture supernatant of S19201R antibody-producing hybridoma and the glycan-related compounds were dispensed were washed with 400 µL of PBST three times and a 5000-fold dilution of HRP-labelled rat IgG (H&L) diluted with BSA-PBST was dispensed by 50 µL/well therein, and left stand at room temperature for one hour. Into the wells in which BSA-PBST was dispensed by 50 µL/well among the wells, the mixture solutions of HRP-labelled DUPAN-2 antibody and the glycan-related compounds were dispensed by 50 µL/well, and left stand at room temperature for one hour.

After each well was washed three times with 400 µL of PBST, 50 µL of citrate buffer solution (pH 5.0) containing 0.2% o-phenylenediamine and 0.02% hydrogen peroxide was added therein and left stand at room temperature for 10 min. After that, 50 µL of 1.5 N sulfuric acid was added therein to stop enzyme reaction, and absorbance at wavelength of 492 nm was measured.

### 2. Experiment Result

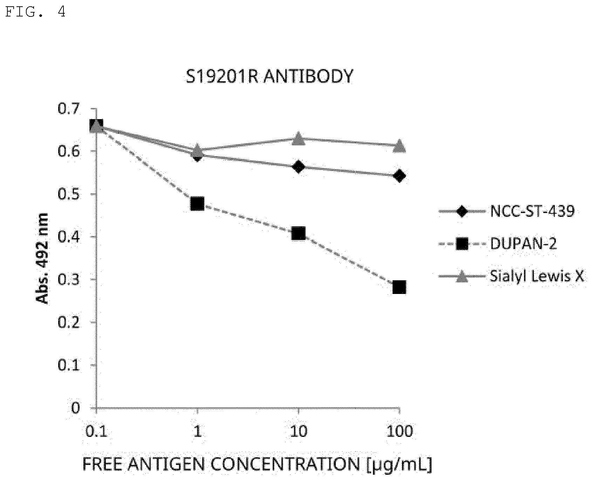

The results are shown in Fig. 4. In case where the conjugates of NCC-ST-439 and DUPAN-2 were mixed in and S19201R antibody was used, a decrease in absorbance was observed only for DUPAN-2 (Fig. 4). In this measurement system, the absorbance depends on an amount of the antibody bound with the conjugate of the DUPAN-2 immobilized on the plate and BSA. That is, the decrease in absorbance as a result of the addition of a glycan-related compound indicates that the glycan-related compound free in the solution reacts with the antibody in the solution and hinders the binding of the antibody and the immobilized conjugate.

### [Experiment Example 4] Reactivity against Sample and Standard Product

### 1. Materials

1. HRP-labelled Streptavidin (Made by Thermo Fisher, 21126)

### 2. Experiment Method

By sandwich ELISA using S19201R antibody, whether or not it was possible to detect DUPAN-2 contained in a blood serum sample was tested. Details of the sandwich ELISA are as below.

### (2-1) Preparation of Plates for Sandwich ELISA

A S19201R antibody-containing solution was dissolved in a 20 mM phosphate buffer solution including 150 mM sodium chloride (pH 7.2; hereinafter, which is referred to as PBS) to attain a concentration of 5 µg/mL, and 50 µL of the solution thus prepared was dispensed in each well of 96-well microplate, and left stand at room temperature for 2 hours.

After each well was washed three times with 400 µL of PBS containing 0.05% Tween (registered trademark) 20 (hereinafter, which is referred to as PBST), 100 µL of PBST containing 1% bovine serum albumin (hereinafter, which is referred to as BSA-PBST) was added therein, and blocking was carried out at room temperature for 1 hour. The plate thus prepared was used as a plate for ELISA.

### (2-2) Sandwich ELISA Method

After each well of the plate of ELISA was washed with 400 µL of PBST three times, 50 µL of a 6-fold dilution or a 12-fold dilution of blood serums of 12 cancer patients diluted with BSA-PBST and gradual dilutions of DUPAN-2 standard product diluted with BSA-PBST were added in the wells and left stand at room temperature for one hour. After each well was washed with 400 µL of PBST three times, 50 µL of S19201R diluted with BSA-PBST to 2 µg/mL and labelled with biotin was dispensed in each well, and left stand at room temperature for one hour. After each well was washed with 400 µL of PBST three times, 50 µL of HRP-labelled Streptavidin diluted with BSA-PBST to 0.2 µg/mL was dispensed in each well, and left stand at room temperature for one hour. After each well was washed three times with 400 µL of PBST, 50 µL of citrate buffer solution (pH 5.0) containing 0.2% o-phenylenediamine and 0.02% hydrogen peroxide was added therein and let stand at room temperature for 10 min. After that, 50 µL of 1.5 N sulfuric acid was added therein to stop enzyme reaction, and absorbance at wavelength of 492 nm was measured. In case where the blood serum of the cancer patients was used, DUPAN-2 concentration was measured, using, as a calibration curve, the absorbance measured by using the standard product.

### 3. Experiment Results

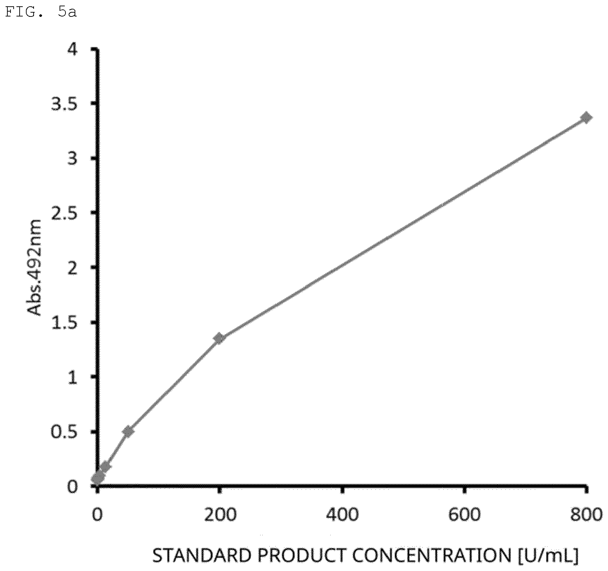

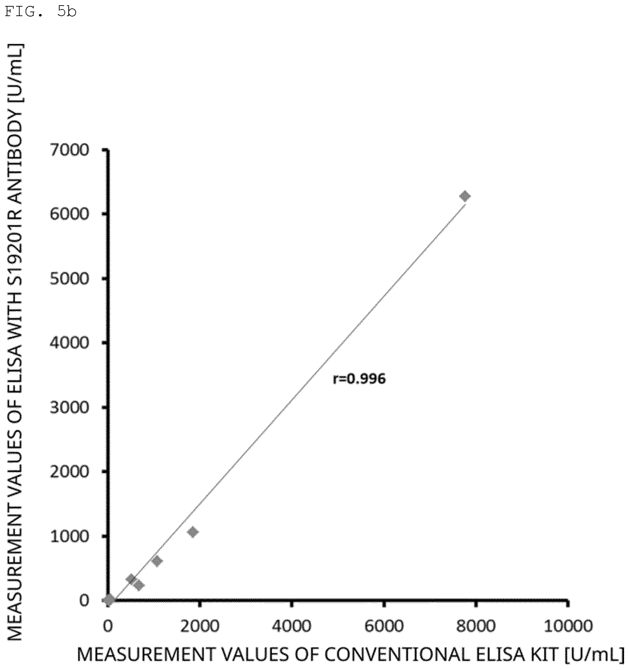

Results are shown in Figs. 5a and 5b. By the sandwich ELISA using S19201R antibody, it was confirmed that the absorbance increased dependently on the concentration of the standard product (Fig. 5a). That is, this demonstrates that S19201R antibody reacted with DUPAN-2 antigen in the standard product. Further, a correlation coefficient of an approximation straight line between a DUPAN-2 concentration in the blood serum of cancer patients measured by a conventional ELISA kit and a DUPAN-2 concentration in the blood serum of cancer patients measured by sandwich ELISA using S19201R antibody was 0.99 or more (Fig. 5b). That is, this demonstrates that S19201R antibody can measure DUPAN-2 in the blood serum of cancer patients as the conventional DUPAN-2 antibody does.

Even though the present invention has been described referring to the concreate Examples, but the scope of the present invention is not limited to these concrete Examples. A person skilled in the art will understand that various modifications can be adopted within the gist of the present invention.

## Claims

1. An antibody or an antigen-binding fragment thereof,
which reacts with DUPAN-2 antigen having a glycan structure below:
Neu5Acαt2-3Galβ1-3GLcNAcβ1-3Gal-
and
does not react with NCC-ST-439 antigen having a structure below:

Neu5Acα2－3Galβ1
4
GlcNAcβ1－6GalNAc－
3
Fucα1
.

2. The antibody or the antigen-binding fragment thereof according to claim 1, wherein reactivity against NCC-ST-439 antigen is less than 10% of reactivity against DUPAN-2 antigen.

3. The antibody or the antigen-binding fragment thereof according to claim 1, wherein reactivity against NCC-ST-439 antigen is less than 1% of reactivity against DUPAN-2 antigen.

4. The antibody or the antigen-binding fragment thereof according to any one of claims 1 to 3, being a rat antibody.

5. A method of producing the antibody or the antigen-binding fragment thereof according to any one of claims 1 to 4, the method comprising:
a step of inoculating an animal with a polymer compound bound with DUPAN-2 antigen.

6. A method of producing cells for producing the antibody or the antigen-binding fragment thereof according to any one of claims 1 to 4, the method comprising:
a step of inoculating an animal with a polymer compound bound with DUPAN-2 antigen.

7. The method according to claim 5 or 6, wherein the polymer compound bound with DUPAN-2 antigen does not contain a peptide portion of DUPAN-2 antigen-binding peptide.

8. An immunity measuring method, wherein the antibody or the antigen-binding fragment thereof according to any one of claims 1 to 3 is employed.

9. An immunity measuring reagent comprising the antibody or the antigen-binding fragment thereof according to any one of claims 1 to 3.
